(19) Europäisches Patentamt
European Patent Office
Office européen des brevets

(11) **EP 2 978 837 B1**

(12) **FASCICULE DE BREVET EUROPEEN**

(45) Date de publication et mention de la délivrance du brevet:
**18.10.2017 Bulletin 2017/42**

(51) Int Cl.:
***C12N 1/12*** *(2006.01)*   ***C12P 21/00*** *(2006.01)*

(21) Numéro de dépôt: **14713448.0**

(22) Date de dépôt: **27.03.2014**

(86) Numéro de dépôt international:
**PCT/EP2014/056125**

(87) Numéro de publication internationale:
**WO 2014/154787 (02.10.2014 Gazette 2014/40)**

(54) **PROCÉDÉ D'ENRICHISSEMENT EN PROTÉINES DE LA BIOMASSE DE MICROALGUES**

VERFAHREN ZUR MIKROALGENBIOMASSEPROTEINANREICHERUNG

MICROALGAL BIOMASS PROTEIN ENRICHMENT METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.03.2013 FR 1352857**
**19.08.2013 FR 1358052**

(43) Date de publication de la demande:
**03.02.2016 Bulletin 2016/05**

(60) Demande divisionnaire:
**16168232.3 / 3 067 414**

(73) Titulaire: **Roquette Frères**
**62136 Lestrem (FR)**

(72) Inventeurs:
• **MACQUART, Gabriel**
**62350 Mont Bernanchon (FR)**
• **DELAROCHE, Sylvain**
**62219 Longuenesse (FR)**
• **LE RUYET, Marie**
**62400 Bethune (FR)**
• **SEGUEILHA, Laurent**
**59520 Marquette Lez Lille (FR)**

(74) Mandataire: **Gallois, Valérie**
**Cabinet Becker & Associés**
**25, rue Louis le Grand**
**75002 Paris (FR)**

(56) Documents cités:
• **SANSAWA H ET AL: "Production of intracellular phytochemicals in Chlorella under heterotrophic conditions", JOURNAL OF BIOSCIENCE AND BIOENGINEERING, ELSEVIER, AMSTERDAM, NL, vol. 98, no. 6, 1 janvier 2004 (2004-01-01), pages 437-444, XP004727088, ISSN: 1389-1723**
• **DOUCHA JIRI ET AL: "Production of high-density Chlorella culture grown in fermenters", JOURNAL OF APPLIED PHYCOLOGY, vol. 24, no. 1, janvier 2012 (2012-01), pages 35-43, XP002717867, ISSN: 0921-8971**
• **JI YONGCHENG ET AL: "Differential effects of phosphorus limitation on cellular metals in Chlorella and Microcystis", LIMNOLOGY AND OCEANOGRAPHY, vol. 53, no. 5, septembre 2008 (2008-09), pages 1790-1804, XP002729306, ISSN: 0024-3590**

EP 2 978 837 B1

**Description**

La présente invention se rapporte à un procédé d'enrichissement en protéines de la biomasse de microalgues, plus particulièrement du genre *Chlorella,* plus particulièrement encore de l'espèce *Chlorella sorokiniana* ou *Chlorella protothecoides.*

Les algues, macro et micro, ont une richesse spécifique en grande partie inexplorée. Leur exploitation à des fins alimentaire, chimique ou bioénergétique est encore très marginale. Elles recèlent cependant des composants de grande valeur, que seule la faune marine, qui s'en nourrit, n'est véritablement capable d'en apprécier la richesse et l'abondance.

Les microalgues sont en effet des sources de vitamines, lipides, protéines, sucres, pigments et antioxydants.

Les algues et microalgues intéressent ainsi le secteur industriel qui les utilise pour la fabrication de compléments alimentaires, d'aliments fonctionnels, de cosmétiques, de médicaments ou pour l'aquaculture.

Les microalgues sont avant tout des microorganismes photosynthétiques qui colonisent tous les biotopes exposés à la lumière.

A l'échelle industrielle, leur culture monoclonale est réalisée dans des photobioréacteurs (conditions autotrophiques : à la lumière avec du $CO_2$) ou pour certaines d'entre elles, également dans des fermenteurs (conditions hétérotrophiques : à l'obscurité en présence d'une source carbonée).

Quelques espèces de microalgues sont en effet capables de pousser en absence de lumière : *Chlorella, Nitzschia, Cyclotella, Tetraselmis, Crypthecodinium, Schizochytrium.*

Il est d'ailleurs estimé que la culture en conditions hétérotrophiques coûte 10 fois moins chère qu'en conditions phototrophiques car, pour l'Homme du métier, ces conditions hétérotrophiques autorisent :

- l'utilisation de fermenteurs identiques à ceux utilisés pour les bactéries et les levures et permettent le contrôle de tous les paramètres de culture.
- la production de biomasses en quantité bien plus élevée que ce qui est obtenu par culture basée sur la lumière.

L'exploitation rentable des microalgues nécessite généralement la maîtrise des conditions de fermentation permettant d'accumuler ses composants d'intérêt, tels que :

- les pigments (chlorophylle a, b et c, ß-carotène, astaxanthine, lutéine, phycocyanine, xanthophylles, phycoérythrine...) dont la demande est croissante, tant pour leurs remarquables propriétés antioxydantes, que pour leur apport de couleurs naturelles dans l'alimentation,
- les protéines, ceci afin d'en optimiser les qualités nutritives ; ou
- les lipides, ceci afin d'optimiser leur contenu en acides gras (jusqu'à 60 %, voir 80 % en poids de leur matière sèche) notamment pour :

  • les applications biocarburants, mais aussi
  • les applications en Alimentation humaine ou animale, lorsque les microalgues sélectionnées produisent des acides gras polyinsaturés ou PUFAs dits "essentiels" (i.e. apportés par l'alimentation car ne sont pas naturellement produits par l'homme ou l'animal).

Pour parvenir à ce résultat, de premiers procédés de fermentation permettant d'obtenir de hautes densités cellulaires (acronyme anglais : HCD pour *High-Cell-Density*) ont été ainsi beaucoup travaillés, de manière à obtenir des rendements et productivités maximales en protéines ou en lipides.

L'objectif de ces cultures HCD était l'obtention de la concentration la plus élevée possible du produit souhaité dans le laps de temps le plus court.

Ce précepte se vérifie par exemple pour la biosynthèse d'astaxanthine par *Chlorella zofingiensis,* où la croissance de la microalgue s'est avérée corrélée directement avec la production de ce composé (Wang and Peng, 2008, World J Microbiol. Biotechnol., 24(9), 1915-1922).

Cependant, le fait de maintenir la croissance à son taux maximal ($\mu$, en $h^{-1}$) n'est pas toujours corrélé avec la production élevée du produit souhaité.

Il est en effet vite apparu aux spécialistes du domaine qu'il faut par exemple soumettre les microalgues à un stress nutritionnel qui limite leur croissance lorsqu'on souhaite leur faire produire d'importantes réserves lipidiques.

Il est donc procédé désormais au découplage croissance / production dans les procédés fermentaires.

Par exemple, pour favoriser l'accumulation d'acides gras polyinsaturés (ici l'acide docosahexaénoïque ou DHA), la demande de brevet WO 01/54510 recommande de dissocier la croissance cellulaire et la production d'acides gras polyinsaturés.

Chez la microalgue *Schizochytrium sp* souche ATCC 20888, il est ainsi procédé à une première phase de croissance sans limitation en oxygène, de manière à favoriser l'obtention d'une haute densité cellulaire (plus de 100 g/l) puis, dans une deuxième phase, de ralentir progressivement l'apport en oxygène, afin de stresser la microalgue, ralentir sa croissance et enclencher la production des acides gras d'intérêt.

Chez la microalgue *Crypthecodinium cohnii,* la teneur la plus élevée en acide docosahexaénoïque (DHA, acide gras polyinsaturé) est obtenue à faible concentration en glucose (de l'ordre de 5 g/l), et ainsi à faible taux de croissance (Jiang and Chen, 2000, Process Biochem., 35(10), 1205-1209).

Ces résultats illustrent bien le fait que les cinétiques de formation des produits peuvent aussi bien être associées de façon positive que de façon négative avec la croissance des microalgues, voire même avec une combinaison des deux.

De ce fait, dans les cas où la formation des produits n'est pas corrélée avec une croissance cellulaire élevée, il est judicieux de maîtriser le taux de croissance cellulaire.

En général, l'homme du métier choisit de contrôler la croissance des microalgues par la maîtrise des conditions de fermentation (Tp, pH...), ou par l'alimentation régulée en composants nutritionnels du milieu de fermentation (conditions semi continues dites « fed-batch »).

S'il choisit de contrôler la croissance des microalgues en hétérotrophie par l'apport en sources carbonées, l'homme du métier choisit généralement d'adapter la source carbonée (glucose pur, acétate, éthanol...) à la microalgue (*C. cohnii, Euglena gracilis...*) en fonction du métabolite produit (par exemple un acide gras polyinsaturé de type DHA).

La température peut-être également un paramètre clef :

- il a par exemple était rapporté que la synthèse d'acides gras polyinsaturés chez certaines espèces de microalgues, tel que l'EPA par *Chlorella minutissima,* est favorisée à une plus basse température que celle requise pour la croissance optimale de ladite microalgue;
- au contraire le rendement en lutéine est plus élevé chez *Chlorella protothecoides* cultivée en hétérotrophie, lorsque l'on augmente la température de production de 24 à 35°C.

*Chlorella protothecoides* est justement reconnue comme une des meilleures microalgues productrices d'huile.

En conditions hétérotrophiques, elle transforme rapidement les hydrates de carbone en triglycérides (plus de 50 % de sa matière sèche).

Pour optimiser cette production en triglycérides, l'homme du métier est amené à optimiser le flux carboné vers la production d'huile, en agissant sur l'environnement nutritionnel du milieu de fermentation.

Il est ainsi connu que l'accumulation en huile se produit lors d'un apport carboné suffisant, mais dans des conditions de carence en azote.

Le rapport C/N est donc ici déterminant, et il est admis que les meilleurs résultats sont obtenus en agissant directement sur la teneur en azote, la teneur en glucose n'étant pas limitante.

De manière non surprenante, cette carence en azote affecte la croissance cellulaire, ce qui résulte en un taux de croissance de 30 % plus faible que le taux de croissance normal de la microalgue (Xiong et al., Plant Physiology, 2010, 154, pp1001-1011).

Pour expliquer ce résultat, Xiong et al, dans l'article cité supra, démontrent en effet que si l'on divise la biomasse de *Chlorella* en ses 5 composants principaux, i.e. hydrates de carbone, lipides, protéines, ADN et ARN (représentant 85 % de sa matière sèche), si le rapport C/N n'a aucun impact sur la teneur en ADN, ARN et hydrates de carbone, il devient prééminent pour le contenu en protéines et en lipides.

C'est ainsi que les cellules de Chlorelles cultivées avec un rapport C/N faible contiennent 25,8 % de protéines et 25,23 % en lipides, tandis qu'un rapport C/N élevé permet la synthèse de 53,8 % de lipides et 10,5 % de protéines.

Pour optimiser sa production en huile, il est donc primordial pour l'homme du métier de contrôler le flux carboné en le déviant vers la production d'huile, au détriment de la production des protéines ; le flux carboné est redistribué et s'accumule en substances de réserve lipidiques quand les microalgues sont placées en milieu carencé en azote.

Fort de cet enseignement, pour la production de biomasses riches en protéines, l'homme du métier est donc amené à prendre l'opposé de ce contrôle métabolique, i.e. travailler les conditions de fermentation en favorisant plutôt un rapport C/N faible, et ainsi :

- réaliser un apport important en source d'azote au milieu de fermentation, tout en maintenant constant la charge en source carbonée qui sera convertie en protéines et
- stimuler la croissance de la microalgue.

Il s'agit de modifier le flux carboné vers la production de protéines (et donc de biomasses), au détriment de la production des lipides de réserve.

SANSAWA et al. (2004, Journal of Bioscience and Bioengineering, 98, 437-444) décrit un procédé de culture en hétérotrophie d'une microalgue, *Chlorella regularis*, comportant une étape visant à limiter la croissance de ladite microalgue par une carence totale d'alimentation en glucose du milieu de fermentation, permettant ainsi d'atteindre une teneur en protéines de la biomasse de 63 % en poids.

DOUCHA et al. (2011, Journal of Applied Phycology, 24, 35-43) décrit un procédé de culture hétérotrophique d'une microalgue, *Chlorella vulgaris,* permettant d'atteindre une teneur en protéines de la biomasse de 36,5 % en poids. La stratégie utilisée consiste à soumettre la biomasse produite à une carence totale de glucose pendant 12h.

Dans le cadre de l'invention, la société Demanderesse a choisi d'explorer une voie originale en proposant une solution alternative à celle classiquement envisagée par l'homme du métier.

L'invention porte ainsi sur un procédé d'enrichissement en protéines d'une microalgue cultivée en hétérotrophie, microalgue du genre *Chlorella,* plus particulièrement encore *Chlorella sorokiniana* ou *Chlorella protothecoides,* procédé de culture hétérotrophique qui comporte une étape visant à limiter la croissance de ladite microalgue par une carence en glucose du milieu de fermentation.

Cette étape est une étape de culture hétérotrophique où le glucose est apporté en quantité insuffisante dans le milieu pour permettre la croissance de la microalgue. Il est à noter que par quantité insuffisante n'est pas entendu un apport nul en ce facteur nutritif. Cette phase de carence nutritive conduit alors à ralentir (limiter) le métabolisme cellulaire, sans l'inhiber totalement.

Au sens de l'invention, « l'enrichissement » s'entend d'une augmentation de la teneur en protéines de la biomasse d'au moins 15 %, de préférence d'au moins 20 % en poids, de manière à atteindre une teneur en protéines de la biomasse de plus de 50 % en poids.

L'invention couvre plus précisément un procédé de culture hétérotrophique desdites microalgues comportant une étape visant à limiter la croissance de ladite microalgue par une carence du milieu de fermentation en glucose.

Ainsi la présente invention est relative à un procédé d'enrichissement en protéines d'une microalgue cultivée en hétérotrophie, microalgue du genre *Chlorella,* plus particulièrement encore *Chlorella sorokiniana* ou *Chlorella protothecoides,* le procédé comprenant la culture hétérotrophique qui comporte une étape visant à limiter la croissance de ladite microalgue par une carence du milieu de fermentation en glucose avec un apport continu en glucose de manière à obtenir une vitesse de croissance comprise entre 0,06 h-1 et 0,09 h-1, permettant ainsi d'atteindre une teneur en protéines de la biomasse de plus de 50 % en poids.

Par « carence du milieu de fermentation en glucose » on entend une culture dans laquelle au moins le glucose est apporté à la microalgue en quantité insuffisante pour permettre sa croissance.

Notamment, le glucose est carencé de sorte à obtenir une vitesse de croissance diminuée de 10 à 60 % par rapport à la vitesse de croissance sans limitation de ladite source nutritionnelle. Il est notamment proposé de diminuer la vitesse de croissance de 10 à 60 % par rapport à la vitesse de croissance sans limitation en glucose, notamment de 10, 15, 20, 25, 30, 35, 40, 45, 50 ou 55 % par rapport à la vitesse de croissance sans limitation en glucose. De préférence, la vitesse de croissance est diminuée de 15 à 55 %.

La durée de la phase de culture carencée en glucose est au moins 1 h, de préférence d'au moins 10 h, plus préférentiellement d'au moins 20 h, notamment entre 30 et 60 h.

Cela se traduit par une absence de glucose résiduel dans le milieu de culture, la microalgue consommant ce glucose au fur et à mesure de son apport. Cependant, l'absence de glucose résiduel dans le milieu de culture est à distinguer d'une situation dans laquelle la microalgue est totalement privée du glucose.

Au sens de l'invention, le critère essentiel est donc bien la limitation de la croissance cellulaire induite par un stress, stress cellulaire provoquée par la carence du glucose du milieu de fermentation.

Cette stratégie va donc bien à l'encontre du préjugé technique selon lequel pour augmenter la teneur en protéines de la biomasse, il faut immanquablement augmenter cette biomasse et donc la croissance cellulaire.

Comme il sera exemplifié ci-après, on peut choisir avantageusement de limiter la croissance de *Chlorella sorokiniana* par une carence en glucose du milieu de fermentation.

Le glucose est apporté de manière à obtenir une vitesse de croissance comprise entre 0,06 h-1 et 0,09 h-1.

Dans un mode très particulier, la souche de *Chlorella sorokiniana* est la souche UTEX 1663 - *The Culture Collection of Algae at the University of Texas at Austin* - USA. Dans un mode très particulier, la souche de *Chlorella protothecoides* est la souche CCAP211/8D - *The Culture Collection of Algae and Protozoa, Scottand, UK.*

De manière optionnelle, la limitation de la croissance de ladite microalgue peut être obtenue par l'ajout dans le milieu de culture de substances inhibitrices de la croissance cellulaire, comme les sulfates.

Par ailleurs, sans être liée par une quelconque théorie, la société Demanderesse a trouvé que le flux de glucose est normalement utilisé chez les microalgues du genre *Chlorella sorokiniana* selon un ordre de priorité bien précis :

  1. le métabolisme basal,
  2. la croissance, i.e. formation d'une biomasse riche en protéines,
  3. les substances de réserve (lipides et hydrates de carbone tel l'amidon).

Ce principe permet d'expliquer les variations naturelles de teneur en protéines au cours de la croissance de la microalgue, malgré l'apport constant en azote.

La société Demanderesse a ainsi trouvé que, pour enrichir la biomasse de microalgues en protéines, il faut limiter la croissance de la microalgue et contrôler sa consommation en source nutritive autre que l'azote, par exemple en glucose de manière à:

- dédier la consommation en glucose toute entière aux voies de production de protéines,
- éviter l'accumulation de substance de réserve comme les lipides.

En effet, éviter une carence en azote permet d'empêcher la déviation des flux métaboliques vers la production de lipides.

De manière optionnelle, il peut être avantageux d'aller jusqu'à bloquer complètement toute synthèse de matériel de réserve, en agissant par l'intermédiaire d'inhibiteurs spécifiques.

Il est connu en effet un certain nombre d'inhibiteurs des voies de synthèse des lipides voire même de l'amidon (hydrate de carbone de réserve par excellence des microalgues vertes) :

- pour les lipides, la cérulénine est décrite comme inhibiteur de la synthèse des acides gras, ou la lipstatine, substance naturelle produite par *Streptomyces toxytricini,* comme inhibiteur des lipases...
- pour l'amidon, les iminosucres (obtenu par simple substitution de l'atome d'oxygène endocyclique des sucres par un atome d'azote) sont historiquement connus en tant qu'inhibiteurs puissants des glycosidases, des glycosyltransférases, des glycogènes phosphorylases, ou de l'UDP-Galp mutase.

Ainsi, le procédé comprend la fermentation d'une biomasse de microalgues en conditions hétérotrophiques avec une première étape de croissance de la biomasse et avec une deuxième étape de carence du milieu de fermentation en glucose.

Cette deuxième étape permet d'enrichir la biomasse en protéine. En particulier, elle permet d'atteindre une teneur en protéines de la biomasse de plus de 50 % en poids (en poids de matière sèche).

Dans un premier mode préféré de réalisation conforme à l'invention, le procédé de culture hétérotrophique desdites microalgues, notamment *Chlorella sorokiniana,* comprend:

° une première étape de croissance des microalgues, où le débit d'apport en glucose est ajusté à leur capacité de consommation, de façon à obtenir rapidement une biomasse importante,

° une deuxième étape de production de protéines par les microalgues, où le débit d'apport en glucose est fixé à une valeur nettement en dessous de leur capacité de consommation du glucose, de façon à empêcher l'accumulation supplémentaire de substances de réserve, voire favoriser leur consommation.

Comme il sera exemplifié ci-après, la première étape de croissance des microalgues est réalisée en mode discontinu ou "batch", dans lequel le glucose apporté initialement est entièrement consommé par la microalgue, conduisant à la production d'une biomasse de base.

La deuxième étape de production de protéines est conduite dans des conditions où on réalise :

- soit un apport de milieu complet en mode semi-continu, dit « fed-batch », après la consommation du glucose apporté initialement ; les autres paramètres de conduite de la fermentation sont inchangés.

Le glucose est apporté en continu et la vitesse d'apport est alors inférieure à la vitesse de consommation que la souche pourrait réaliser de sorte que la teneur résiduelle en glucose dans le milieu est maintenue nulle.

La croissance de la souche est alors limitée par la disponibilité en glucose (condition glucose-limitant).

- un fonctionnement en continu de type chemostat, dans lequel le taux de croissance de la souche ($\mu$) est maintenu à sa valeur minimale, la croissance de la souche étant limitée par l'apport en glucose.

Ce mode de fonctionnement permet d'obtenir une biomasse à forte teneur en protéines grâce à la limitation en glucose et au faible taux de croissance imposé tout en assurant une très bonne productivité.

L'invention sera mieux comprise à l'aide des exemples qui suivent.

**Exemples**

**Exemple 1 : Production de *Chlorella sorokiniana* en fermentation de type « batch séquentiel » sans limitation de l'apport en milieu nutritif**

La souche utilisée est une *Chlorella sorokiniana* (souche UTEX 1663 - *The Culture Collection of Algae at the University of Texas at Austin* - USA).

*Préculture :*

- 600 mL de milieu dans un Erlenmeyer de 2 L ;
- Composition du milieu (tableau 1 ci-dessous)

Tableau 1.

| | | |
|---|---|---|
| Macro éléments (g/L) | Glucose | 20 |
| | $K_2HPO_4.3H2O$ | 0,7 |
| | $MgSO_4.7H2O$ | 0,34 |
| | Acide citrique | 1,0 |
| | Urée | 1,08 |
| | $Na_2SO_4$ | 0,2 |
| | $Na_2CO_3$ | 0,1 |
| | clerol FBA 3107 (antimousse) | 0,5 |
| éléments (mg/l) | $Na_2EDTA$ | 10 |
| | $CaCl_2.2H2O$ | 80 |

| | | |
|---|---|---|
| | $FeSO_4.7H2O$ | 40 |
| | $MnSO_4.4H2O$ | 0,41 |
| | $CoSO_4.7H2O$ | 0,24 |
| | $CuSO_4.5H2O$ | 0,24 |
| | $ZnSO_4.7H2O$ | 0,5 |
| | $H_3BO_3$ | 0,11 |
| | $(NH_4)_6Mo_7O_{27}.4H2O$ | 0,04 |

Le pH est ajusté à 7 avant stérilisation par ajout de NaOH 8N.

L'incubation se déroule dans les conditions suivantes :

- durée : 72 h ;
- température : 28°C ;
- agitation : 110 rpm (Incubateur Infors Multitron).

La préculture est ensuite transférée dans un fermenteur de 30L de type Sartorius.

*Culture pour production de biomasse :*

Le milieu est identique à celui de la préculture, mais l'urée est remplacée par du $NH_4Cl$.

Tableau 2.

| | | | |
|---|---|---|---|
| Macro éléments (g/L) | Glucose | 20 | |
| | K$_2$HPO$_4$.3H2O | 0,7 | |
| | MgSO$_4$.7H2O | 0,34 | |
| | Ac Citrique | 1,0 | |
| | NH$_4$Cl | 1,88 | |
| | Na$_2$SO$_4$ | 0,2 | |
| | clerol FBA 3107 (antimousse) | 0,5 | |
| Micro éléments (mg/L) | Na$_2$EDTA | 10 | |
| | CaCl$_2$ | 80 | |
| | FeSO$_4$.7H2O | 40 | |
| | MnSO$_4$.4H2O | 0,41 | |
| | CoSO$_4$.7H2O | 0,24 | |
| | CuSO$_4$.5H2O | 0,24 | |
| | ZnSO$_4$.7H2O | 0,5 | |
| | H$_3$BO$_3$ | 0,11 | |
| | (NH$_4$)$_6$Mo$_7$O$_{27}$.4H2O | 0,04 | |

Le volume initial (Vi) du fermenteur est ajusté à 13,5 L après ensemencement.

Il est porté à 16 - 20 L en final.

Les paramètres de conduite de la fermentation sont les suivants :

Tableau 3.

| Température | 28 °C |
|---|---|
| pH | 5,0 - 5,2 par NH$_3$ 28% p/p |
| pO$_2$ | > 20% (maintenue par agitation) |
| Agitation | 300 RPM mini |
| Débit d'air | 15 L/min |

Lorsque le glucose apporté initialement est consommé, un apport de milieu identique au milieu initial, sans l'antimousse, est réalisé sous forme d'une solution concentrée contenant 500 g/L de glucose et les autres éléments dans les mêmes proportions par rapport au glucose que dans le milieu initial, de façon à obtenir une teneur en glucose de 20 g/L dans le fermenteur.

Deux autres ajouts identiques sont réalisés de la même manière à chaque fois que la concentration résiduelle en glucose devient nulle.

De l'antimousse Clerol FBA 3107 est ajouté à la demande pour éviter un moussage excessif.

Résultats :

Après 46 h de culture, on obtient 38 g/L de biomasse à une teneur en protéines (évalué par le N 6,25) de 36,2 %

**Exemple 2 : Production de *C. sorokiniana* en fermentation de type « fed batch » avec apport en glucose limitant**

Dans cet exemple, un apport de milieu complet (mode « fed-batch ») est démarré après la consommation du glucose apporté initialement. Les autres paramètres de conduite de la fermentation sont inchangés.

Le glucose est apporté en continu à l'aide d'une solution concentrée à 500 g/L. La vitesse d'apport est inférieure à la vitesse de consommation que la souche pourrait réaliser de sorte, que la teneur résiduelle en glucose dans le milieu est maintenue nulle, c'est-à-dire que la croissance de la souche est limitée par la disponibilité en glucose (condition glucose-limitant).

Cette vitesse est augmentée au cours du temps de façon exponentielle. La formule servant à calculer le débit d'ajout est caractérisée par un facteur μ qui correspond au taux de croissance que la souche peut adopter si elle consomme tout le glucose fourni :

$$S = So \times exp\ (\mu \cdot t)$$

S = débit d'apport en glucose (en g/h)
So = débit d'apport initial en glucose, déterminé en fonction de la biomasse présente en fin de batch. Il est de 12 g/h dans nos conditions.
μ = facteur d'accélération du débit. Il doit être inférieur à 0,11 h⁻¹ qui est le taux de croissance de la souche en absence de limitation nutritionnelle.
t = durée du fed-batch (en h)

Les sels sont apportés si possible en continu, séparément ou en mélange avec le glucose. Mais ils peuvent être aussi apportés séquentiellement en plusieurs fois.

Le tableau 4 ci-dessous donne les besoins en sels pour 100 g de glucose.

Tableau 4.

| | | |
|---|---|---|
| Macro éléments (g) | Glucose | 100 |
| | $K_2HPO_4.3H2O$ | 6,75 |
| | $MgSO_4.7H2O$ | 1,7 |
| | Ac Citrique | 5,0 |
| | Na2SO4 | 1,0 |
| Micro éléments (mg) | $Na_2EDTA$ | 50 |
| | $CaCl_2.2H2O$ | 400 |
| | $FeSO_4.7H2O$ | 200 |
| | $MnSO_4.4H2O$ | 2,1 |
| | $CoSO_4.7H2O$ | 1,2 |
| | $CuSO_4.5H2O$ | 1,2 |
| | $ZnSO_4.7H2O$ | 2,5 |
| | $H_3BO_3$ | 0,6 |
| | $(NH_4)_6Mo_7O_{27}.4H2O$ | 0,2 |

Les concentrations des autres éléments que le glucose ont été déterminées de façon à être en excès par rapport aux besoins nutritionnels de la souche.

De l'antimousse Clerol FBA 3107 est ajouté à la demande pour éviter un moussage excessif.

Résultats : effet de la vitesse d'apport en glucose durant le fed-batch

Des essais ont été effectués à différentes vitesses d'apport du glucose en « fed-batch ». Ils sont caractérisés par le μ appliqué. La teneur en protéine de la biomasse obtenue est évaluée par la mesure de l'azote total exprimée en N 6.25.

Tableau 5.

| Essai | μ fed (h-1) | Durée (h) | Biomasse (g/L) | Productivité (g/L/h) | % N 6.25 |
|---|---|---|---|---|---|
| 1 | 0,06 | 78 | 43,6 | 0,56 | 49,2 |
| 2 | 0,07 | 54 | 35,1 | 0,65 | 43,1 |

(suite)

| Essai | $\mu$ fed (h-1) | Durée (h) | Biomasse (g/L) | Productivité (g/L/h) | % N 6.25 |
|-------|-------|-------|-------|-------|-------|
| 3 | 0,09 | 48 | 64,9 | 1,35 | 39,3 |

Ces résultats montrent que le fait de travailler à glucose limitant permet d'augmenter la teneur en protéines.

Il est en effet observé que, même avec un $\mu$ élevé de 0,09, on obtient une teneur en protéines plus élevée que celle obtenue sans limitation comme dans l'exemple 1 (39,3 % au lieu de 36,2 %).

Accentuer la limitation du métabolisme par le glucose entraine une amélioration supplémentaire de la teneur en protéines.

Dans les conditions de ces essais, il est nécessaire d'imposer à la souche un $\mu$ inférieur à 0,06 h-1 pour obtenir une teneur en protéines supérieure à 50%.

Il est à noter que cette condition va de pair avec une réduction de la productivité : 0,56 g/Uh au lieu de 1,35 g/L/h avec l'essai 3.

**Exemple 3 : Production de *C. sorokiniana* en fermentation continue de type chemostat avec apport de glucose limitant**

Dans cet exemple, le fermenteur utilisé est un 2L de type Sartorius Biostat B.

Il est réalisé comme l'exemple 2, mais avec des volumes 10 fois plus faibles : l'inoculum est de 60 ml et le volume initial de 1,35L.

L'apport en continu du milieu est démarré selon le même principe que dans l'exemple 2, les sels étant dans ce cas mélangés avec le glucose dans la nourrice d'alimentation. La vitesse d'apport est accélérée selon la même formule exponentielle que dans l'exemple 2 en appliquant un $\mu$ de 0,06 h-1.

Chemostat

Lorsqu'un volume de 1,6 L est atteint, soit une concentration en biomasse d'environ 50 g/L, le fonctionnement en continu de type chemostat est mis en place :

1. Le fermenteur est alimenté en continu à un débit de 96 ml/h par une solution de milieu nutritif à 100 g/L de glucose de composition suivante :

Tableau 6.

| | | |
|---|---|---|
| Macro éléments (g/L) | Glucose | 100 |
| | $K_2HPO_4.3H2O$ | 6,75 |
| | $MgSO_4.7H2O$ | 1,7 |
| | Ac Citrique | 5,0 |
| | Na2SO4 | 1,0 |
| Micro éléments (mg/L) | $Na_2EDTA$ | 50 |
| | $CaCl_2.2H2O$ | 400 |
| | $FeSO_4.7H2O$ | 200 |
| | $MnSO_4.4H2O$ | 2,1 |
| | $CoSO_4.7H2O$ | 1,2 |
| | $CuSO_4.5H2O$ | 1,2 |
| | $ZnSO_4.7H2O$ | 2,5 |
| | $H_3BO_3$ | 0,6 |
| | $(NH_4)_6Mo_7O_{27}.4H2O$ | 0,2 |

Les concentrations des autres éléments que le glucose ont été déterminé de façon à être en excès par rapport aux besoins nutritionnels de la souche.

2. Du milieu est soutiré en continu du fermenteur grâce à un tube plongeant relié à une pompe de façon à maintenir le volume de la culture à 1,6L.

Ainsi, le milieu est renouvelé d'une fraction 0,06 (6 %) par heure. Ce taux de renouvellement est appelé taux de dilution (D).

Conformément au principe de la culture en chemostat, le taux de croissance de la souche (µ) s'établit à la même valeur car la croissance de la souche est limitée par l'apport en glucose :

$$D = \mu = 0,06 \text{ h}^{-1}$$

Résultats

Après 97 h de fonctionnement en chemostat, la concentration en biomasse se stabilise à 48 g/L +/- 2 g/L et la teneur en protéine à 53 +/- 2 %.

Ce mode de fonctionnement permet d'obtenir une biomasse à forte teneur en protéine grâce à la limitation en glucose et au faible taux de croissance imposé tout en assurant une très bonne productivité, de l'ordre de 2,9 g/L/h, grâce à la concentration élevée en biomasse.

**Revendications**

1. Procédé d'enrichissement en protéines d'une microalgue cultivée en hétérotrophie, microalgue du genre *Chlorella,* **caractérisé en ce que** la culture hétérotrophique comporte une étape visant à limiter la croissance de ladite microalgue par une carence en glucose du milieu de fermentation avec un apport continu en glucose de manière à obtenir une vitesse de croissance comprise entre 0,06 h-1 et 0,09 h-1, permettant ainsi d'atteindre une teneur en protéines de la biomasse de plus de 50 % en poids.

2. Procédé selon la revendication 1, **caractérisé en ce que** le glucose est carencé de sorte à obtenir une vitesse de croissance diminuée de 10 à 60 % par rapport à la vitesse de croissance sans limitation dudit glucose.

3. Procédé selon l'une quelconque des revendications 1 à 2, **caractérisé en ce que** la microalgue du genre Chlorella est choisie dans le groupe constitué de *Chlorella sorokiniana* et *Chlorella protothecoides.*

4. Procédé selon l'une quelconque des revendications 1 à 3, **caractérisé en ce que** la durée de la phase de culture carencée est d'au moins 1 h, de préférence d'au moins 10 h, plus préférentiellement d'au moins 20 h, notamment entre 30 et 60 h.

5. Procédé selon l'une quelconque des revendications 1 à 4, **caractérisé en ce que** la culture hétérotrophique des microalgues de l'espèce *Chlorella sorokiniana* comprend :

a. une première étape de croissance des microalgues, où le débit d'apport en glucose est ajusté à leur capacité de consommation, de façon à obtenir rapidement une biomasse importante,
b. une deuxième étape de production de protéines par les microalgues, où le débit d'apport en glucose est fixé à une valeur nettement en dessous de leur capacité de consommation du glucose, de façon à empêcher l'accumulation supplémentaire de substances de réserve, voire favoriser leur consommation.

**Patentansprüche**

1. Verfahren zur Anreicherung von Proteinen einer heterotroph kultivierten Mikroalge der Gattung *Chlorella,* **dadurch gekennzeichnet, dass** die heterotrophe Kultivierung einen Schritt der Limitierung des Wachstums besagter Mikroalge durch einen Glucose-Mangel des Fermentationsmediums bei einer kontinuierlichen Glucose-Zuführung umfasst, um eine Wachstumsrate zwischen 0,06 h$^{-1}$ und 0,09 h$^{-1}$ zu erhalten, um folglich einen Proteingehalt der Biomasse von mehr als 50 Gew.-% zu erreichen.

**2.** Verfahren gemäß Anspruch 1, **dadurch gekennzeichnet, dass** der Glucose-Mangel zu einer um 10 bis 60% verminderten Wachstumsrate bezogen auf die Wachstumsrate ohne Limitierung besagter Glucose führt.

**3.** Verfahren gemäß einem der Ansprüche 1 bis 2, **dadurch gekennzeichnet, dass** die Mikroalge der Gattung *Chlorella* aus der Gruppe ausgewählt ist, bestehend aus *Chlorella sorokiniana* und *Chlorella protothecoides.*

**4.** Verfahren gemäß einem der Ansprüche 1 bis 3, **dadurch gekennzeichnet, dass** die Dauer der Mangelkultivierungsphase wenigstens 1 h, bevorzugt wenigstens 10 h, bevorzugter wenigstens 20 h, insbesondere zwischen 30 und 60 h beträgt.

**5.** Verfahren gemäß einem der Ansprüche 1 bis 4, **dadurch gekennzeichnet, dass** die heterotrophe Kultivierung der Mikroalgen der Spezies *Chlorella sorokiniana* umfasst:

a) einen ersten Schritt des Wachstums der Mikroalgen, in dem die Zuführungsrate von Glucose an die Verbrauchskapazität der Mikroalgen angepasst ist, um eine große Biomasse schnell zu erhalten,
b) einen zweiten Schritt der Produktion von Proteinen von den Mikroalgen, in dem Zuführungsrate von Glucose auf einen Wert festgesetzt ist, der deutlich unter ihrer Verbrauchskapazität von Glucose liegt, um die zusätzliche Akkumulation von Reservestoffen zu verhindern oder sogar deren Verbrauch zu fördern.

**Claims**

**1.** A method of protein enrichment of a microalga grown heterotrophically, said microalga being of the genus *Chlorella,* **characterized in that** the heterotrophic culturing comprises a step aiming at limiting the growth of said microalga by means of a deficiency of glucose in the fermentation medium with a continuous supply in glucose so as to obtain a growth rate of between 0.06 h$^{-1}$ and 0.09 h$^{-1}$, thus making it possible for the biomass protein content to reach more than 50% by weight.

**2.** The method as claimed in claim 1, **characterized in that** the glucose is deficient so as to obtain a growth rate that is 10% to 60% lower than the growth rate without the limiting of said glucose.

**3.** The method as claimed in any one of claims 1 to 2, **characterized in that** the microalga of the genus *Chlorella* is selected from the group consisting of *Chlorella sorokiniana* and *Chlorella protothecoides.*

**4.** The method as claimed in any one of claims 1 to 3, **characterized in that** the duration of the deficient culturing phase is at least 1 h, preferably at least 10 h, more preferably at least 20 h, in particular between 30 h and 60 h.

**5.** The method as claimed in any one of claims 1 to 4, **characterized in that** the heterotrophic culturing of microalgae of the species *Chlorella sorokiniana* comprises:

a. a first step in which the microalgae are grown, where the glucose supply rate is adjusted to the consumption capacity of said microalgae, so as to obtain a large biomass quickly,
b. a second step in which the microalgae produce proteins, where the glucose supply rate is set to a value distinctly below the glucose consumption capacity of said microalgae, so as to prevent the additional accumulation of storage substances, even to promote their consumption.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0154510 A

**Littérature non-brevet citée dans la description**

- **WANG ; PENG.** *World J Microbiol. Biotechnol.,* 2008, vol. 24 (9), 1915-1922
- **JIANG ; CHEN.** *Process Biochem.,* 2000, vol. 35 (10), 1205-1209
- **XIONG et al.** *Plant Physiology,* 2010, vol. 154, 1001-1011
- **SANSAWA et al.** *Journal of Bioscience and Bioengineering,* 2004, vol. 98, 437-444
- **DOUCHA et al.** *Journal of Applied Phycology,* 2011, vol. 24, 35-43